(19) Europäisches Patentamt
European Patent Office
Office européen des brevets

(11) EP 1 082 099 B1

(12) EUROPEAN PATENT SPECIFICATION

(45) Date of publication and mention of the grant of the patent:
**20.10.2004 Bulletin 2004/43**

(21) Application number: **99922571.7**

(22) Date of filing: **28.05.1999**

(51) Int Cl.[7]: **A61K 7/48**

(86) International application number:
**PCT/JP1999/002858**

(87) International publication number:
**WO 1999/062482 (09.12.1999 Gazette 1999/49)**

# (54) SURFACTANT FOR USE IN EXTERNAL PREPARATIONS FOR SKIN AND EXTERNAL PREPARATION FOR SKIN CONTAINING THE SAME

TENSID FÜR ÄUSSERLICH ANZUWENDENDE HAUTPRÄPARATE UND DIESE BEINHALTENDE ÄUSSERLICH ANZUWENDENDE HAUTPRÄPARATE

TENSIOACTIF POUR PREPARATION A USAGE EXTERNE DE SOIN DE LA PEAU, PREPARATION A USAGE EXTERNE DE SOIN DE LA PEAU CONTENANT CE TENSIOACTIF

(84) Designated Contracting States:
**AT BE CH CY DE DK ES FI FR GB GR IE IT LI LU MC NL PT SE**

(30) Priority: **29.05.1998 JP 14908898**
**16.03.1999 JP 7104899**

(43) Date of publication of application:
**14.03.2001 Bulletin 2001/11**

(73) Proprietor: **Showa Denko K K**
**Tokyo 105-8518 (JP)**

(72) Inventors:
- **YONEDA, Tadashi**
  **Ctl. Res. Lab., Showa Denko K.K.**
  **Chiba-shi Chiba 267-0056 (JP)**
- **KATO, Eiko Ctl. Res. Lab., Showa Denko K.K.**
  **Chiba-shi Chiba 267-0056 (JP)**
- **TSUZUKI, Toshi**
  **Ctl. Res. Lab., Showa Denko K.K.**
  **Chiba-shi Chiba 267-0056 (JP)**
- **FURUYA, Kazuo**
  **Ctl. Res. Lab., Showa Denko K.K.**
  **Chiba-shi Chiba 267-0056 (JP)**
- **TAKAMA, Michihiro Showa Denko K.K.**
  **Tokyo 105-8518 (JP)**
- **MIYOTA, Yoshiaki Kawasaki Works**
  **Kawasaki-shi Kanagawa 210-0865 (JP)**
- **ITO, Shinobu Showa Denko K.K.**
  **Tokyo 105-8518 (JP)**

(74) Representative: **Strehl Schübel-Hopf & Partner**
**Maximilianstrasse 54**
**80538 München (DE)**

(56) References cited:
**FR-A- 2 668 365** **US-A- 4 600 526**

- **CHEMICAL ABSTRACTS, vol. 128, no. 20, 18 May 1998 (1998-05-18) Columbus, Ohio, US; abstract no. 248380, page 1004; XP002115063 & JP 10 059832 A (LION CORP) 3 March 1998 (1998-03-03)**
- **J. Antibiot., vol 39, no. 6, 745-761**
- **J. Bacteriol., vol. 175, no. 20, 6459-6466, 1993**
- **Biochemistry, vol. 17, no. 19, 3992-39996, 1978**
- **J. Bacteriol., vol. 174, no. 6, 1769-1776, 1992**

EP 1 082 099 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

TECHNICAL FILED

The present invention relates to a surfactant for use in external preparations for skin and to an external preparation for skin containing it. More particularly, the present invention relates to a surfactant for use in external preparations for skin having low skin permeability (penetrability) and low irritation, an external preparation for skin, such as cosmetic, containing such a surfactant, and an external preparation for skin, such as a transparent cosmetic, containing such a surfactant and a sequestering agent.

BACKGROUND ART

Hitherto, in external preparations for skin, such as cosmetics, there have been used anionic surfactants composed of aliphatic higher alcohol sulfates, aliphatic higher alcohol phosphates, N-long chain acyl glutaminates, etc., ether type nonionic surfactants, e.g., aliphatic higher alcohol ethylene oxide adducts, nonionic surfactants composed of higher fatty acid and polyhydric alcohols, as emulsifiers, dispersants, solubilizers, etc.

However, skin irritation of these surfactants cannot be said to be sufficiently low to individuals who have allergic constitution, suffering, e.g., pollenosis, atopic dermatitis, etc. so that external preparations for skin containing such have insufficient safety to skin and improvement thereof has been desired.

Further, even if external preparations for skin are prepared using surfactants having sufficiently low irritation to the skin, the external preparations for skin contain in addition to surfactants irritating substances such as salicylic acid, paraben or hexachlorophene as an antiseptic. Therefore, in order to reduce their irritation to the skin, development of low irritation external preparations for skin has been desired.

Known examples of low irritation surfactants include amino acid derivatives. For example, there have been proposed basic amino acid derivatives produced by reacting a glycidyl ether and a basic amino acid (Japanese Patent Application Laid-open No. Hei 9-271655 (European Patent Application Laid-open No.788,832(A1)), and certain water-soluble glycoxide type surfactants as a surfactant having low irritation and alleviating the irritation of other skin-irritating substances (Japanese Patent Application Laid-open No. Hei 9-235587).

Further, with view to prevent glycine derivatives from coloring and deterioration, there have been proposed detergent compositions having blended therein a metal chelating agent and an antioxidant (Japanese Patent Application Laid-open Nos. Hei 9-78085, Hei 9-87673, and Hei 10-237488). However, these surfactants have problems that they are not fully satisfactory in low irritation, they have low effect of reducing irritation by irritating substances other than surfactants, they decrease the surface activity of other surfactants, their deterioration cannot be prevented completely, and so on.

DISCLOSURE OF THE INVENTION

Therefore, an object of the present invention is to provide a surfactant for use in external preparations for skin, having low irritation to the skin.

Another object of the present invention is to provide a surfactant for use in external preparations for skin, not only having low skin irritation to the skin itself but also reducing the irritation of skin-irritating substances.

Further, an object of the present invention is to provide an external preparation for skin, such as a cosmetic, containing the above-described surfactant for use in external preparations for skin.

The present inventors have made intensive research with view to achieving the above-described objects and as a result they have found that when used as a surfactant, a lipopeptide as defined in claim 1 is low in skin penetrability, is low in irritation to the skin and, surprisingly, has an effect of reducing the irritation by skin-irritating substances.

Further, the present inventors have found that existence of a minute amount of an alkaline earth metal such as calcium or magnesium in external preparations for skin containing the above-described surfactant results in that the surfactant and the alkaline earth metal form a water-insoluble salt, which precipitates and make the preparation turbid whereas blending a sequestering agent together with the surfactant in the external preparation for skin prevents the formation of the water-insoluble salt of the surfactant without affecting the low irritation of the surfactant and the effect of reducing the irritation of skin-irritating substances, so that transparency of the preparation can be retained.

Based on these findings, the present invention provides the use of at least one lipopeptide compound represented by formula (2) below

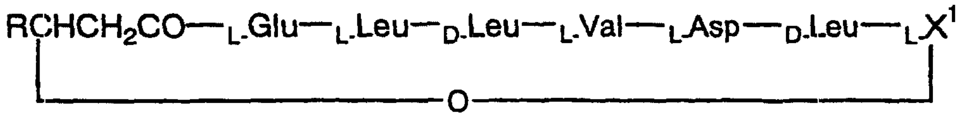

(wherein $X^1$ is an amino acid selected from the group consisting of leucine, isoleucine, valine, glycine, serine, alanine, threonine, asparagine, glutamine, aspartic acid, glutamic acid, lysine, arginine, cystein, methionine, phenylalanine, tyrosine, tryptophan, histidine, proline, 4-hydroxyproline, and homoserine, and R has 9 to 13 carbon atoms and is a n-alkyl group, an isoalkyl group, or an anteisoalkyl group) as a surfactant in external preparations for the skin.

Further preferred embodiments of the invention are defined in claims 2-11.

DETAILED DESCRIPTION OF THE INVENTION

The present invention is based on the discovery that a lipopeptide compound derived from a prokaryote has a low irritation to the skin and reduces the irritation of skin-irritating substances and has applied this to a surfactant for use in external preparations for skin.

Typical examples of the lipopeptide compound derived from a prokaryote includes surfactin. Surfactin is a compound, which is produced usually by a prokaryote, has a lipopeptide structure represented by the formula 1 below.

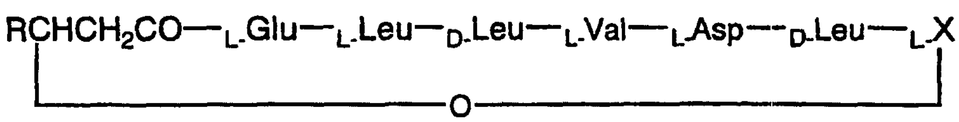

wherein X is leucine, isoleucine or valine, R has 9 to 13 carbon atoms and represents a n-alkyl group, an isoalkyl group or an anteisoalkyl group.

Generally, *Bacillus* microbes are used as the prokaryote. Specific examples thereof include *Bacillus subtilis* IAM 1213 strain, IAM 1069 strain, IAM 1259 strain, IAM 1260 strain, IFO 3035 strain, ATCC 21332 strain, etc.

Surfactin can be obtained without difficulty by cultivating these microbes and purifying lipopeptide compounds that the microbes produced. Purification can be performed, for example, by rendering the culture broth acidic by addition of hydrochloric acid, etc., filtering surfactin which precipitated, dissolving the precipitate in an organic solvent such as methanol, and then optionally practicing ultrafiltration, treatment with activated carbon, crystallization, etc. Precipitation by addition of an acid may be replaced by precipitation by addition of a calcium salt (Biochem. Bioph. Res. Commun., 31: 488-494 (1968)).

Compounds having a lipopeptide structure derived from prokaryotes, other than surfactin, may be used similarly. Examples of such compounds include plipastatin (J. Antibiot., Vol. 39, No. 6, 745-761, 1986), arthrofactin (J. Bacteriol., Vol. 175, No. 20, 6459-6466, 1993), iturin (Biochemistry, Vol. 17, No. 19, 3992-3996, 1978, serrawettin (J. Bacteriol., Vol. 174, No. 6, 1769-1772, 1992).

**Plipastatin**

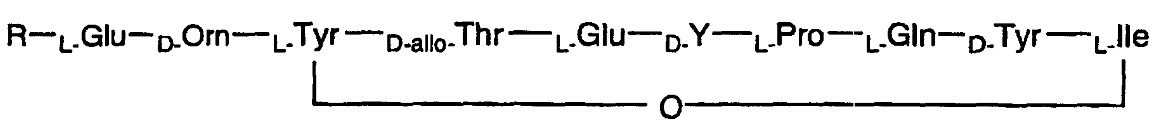

(wherein Y is Val or Ala and Orn stands for ornithine.)

Arthrofactin

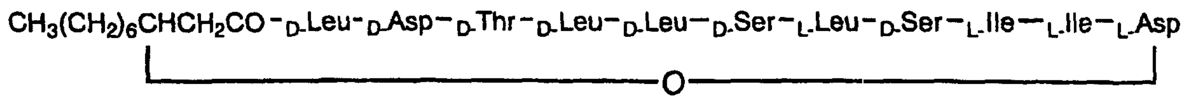

Iturin

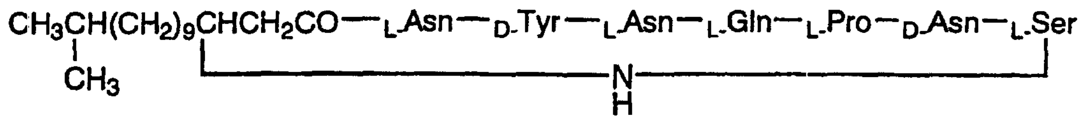

Serrawettin

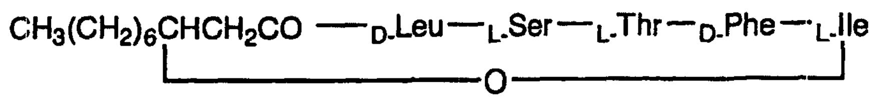

Hereafter, the present invention will be described specifically referring to surfactin as a typical example.

Surfactin generally comprises at least one compound represented by the formula 1 above.

In the formula 1, of the groups having 9 to 13 carbon atoms represented by R, the n-alkyl group is a straight chain alkyl group, the isoalkyl group usually has the structure $(CH_3)_2CH-(CH_2)_n-$, and the anteisoalkyl group usually has the structure $CH_3-CH_2-CH(CH_3)-(CH_2)_n-$.

When surfactin is utilized, the culture broth may be used as it is or purified before it can be used.

As will be apparent from the formula 1, surfactin may be used as a metal salt or organic ammonium salt of a carboxyl group derived from the amino acid structural unit. The metal which serves as a counter ion may be any types of metals so far as they form a salt with surfactin, not to speak of alkali metals such as sodium, potassium, and lithium, alkaline earth metals such as calcium and magnesium. The organic ammonium includes trimethylamine, triethylamine, tributylamine, monoethanolamine, diethanolamine, triethanolamine, lysine, arginine, choline, etc. Among them, preferred are alkali metal salts, particularly sodium salt.

Surfactin shows an anion activity due to the carboxyl group of L-Glu (L-glutamic acid), L-Asp (L-aspartic acid) therein.

The low irritation to the skin of surfactin is considered to be attributable to the fact that surfactin is a cyclic compound of a complexed structure and bulky so that it has a low skin penetrability. Also, surfactin is considered to reduce the irritation of skin-irritating substances because of its masking effect by surrounding the skin-irritating substances.

Further, the present invention provides an external preparation for skin by utilizing surfactin as a surfactant for use in external preparations for skin and blending it together with a sequestering agent to retain transparency.

In the present invention, the surfactant for use in external preparations for skin used has a low skin penetrability and is expected to exhibit the above-described masking effect.

Further, those compounds based on surfactin but with varied amino acid composition, for example, X in the formula 1 is substituted by glycine, serine, alanine, threonine, asparagine, glutamine, aspartic acid, glutamic acid, lysine, arginine, cystein, methionine, phenylalanine, tyrosine, tryptophan, histidine, proline, 4-hydroxyproline, and homoserine, the compound of the formula 2 above, may also be used.

Surfactin and the above-described compounds may also be those obtained by other methods, for example, chemical synthesis, as well as those derived from prokaryotes such as *Bacillus* microbes, and can be used similarly.

The sequestering agent used in the present invention will be explained below.

If metal ions exist in transparent external preparations for skin, they cause deterioration of the quality of external preparations for skin, such as generation of turbidity or precipitation. The sequestering agent is used for the purpose of preventing such.

The sequestering agent which can be used in the present invention may be of any type so far as it has an acidic group having a salt-forming ability or an atomic group having an ability of coordination and can sequester metal ions. Specific examples of the sequestering agent includes L-alanine, DL-alanine, trisodium ethylenediaminehydroxyethyltriacetate, trisodium ethylenediaminehydroxyethyltriasetate dihydrate, edetic acid, dipotassium edetate dihydrate, disodium edetate, disodium calcium edetate, trisodium edetate, tetrasodium edetate, tetrasodium edetate dihydrate, tetrasodium edetate tetrahydrate, sodium citrate, gluconic acid, sodium gluconate, tartaric acid, phytic acid, sodium polyphosphate, sodium metaphosphate, tetrasodium L-glutaminate diacetate, etc.

These may be used singly or two or more of them may be used simultaneously.

Of these, disodium edetate and sodium citrate are particularly preferred.

The sequestering agent forms salts with alkaline earth metals such as calcium and magnesium so that it prevents the formation of salts between surfactin and alkaline earth metals.

The external preparation for skin of the present invention contains the above-described surfactant or the above-described surfactant and sequestering agent. In the external preparations for skin, the surfactant used in the present invention serves as an emulsifier, a dispersant, a solubilizer, a wetting agent, a detergent, a humectant, etc. and also acts as an irritation-reducing agent for skin-irritating substances. There is no limitation on the form in which the surfactant is contained in external preparations for skin, which may be achieved by any one of dissolution, emulsification, dispersion, mixing, etc. and may be in any form such as liquid, milky lotion, gel, solid (inclusive of powder and granules). It may be in the state where vesicles are formed in a solution.

The amount of surfactant in external preparations for skin is generally in a range of 0.01 to 30 wt%. The amount of sequestering agent is generally equivalent to or larger than the amount of the alkaline earth metal contained in the external preparation for skin, which gives sufficient effects. More specifically, it may be used in a range of 0.0001 to 3 wt%, preferably 0.001 to 0.2 wt%.

A typical example of external preparations for skin is a cosmetic. Specific examples thereof include skin milk, skin cream, foundation cream, massaging cream, cleansing cream, shaving cream, cleansing foam, a beauty wash, lotion, pack, shampoo, rinse, a hair restoration agent, a hair tonic, a hair dye, a hair dressing, dentifrice, a gargle, permanent waving agent, ointment, bath additive, body soap, etc. Any type of external preparations for skin may be included so far as it is brought in contact with the skin when in use. Sex and age of users are irrelevant.

The skin-irritating substances whose irritation is reduced by the surfactant for use in external preparations for skin according to the present invention includes antiseptics, ultraviolet absorbents, antioxidants, dyes, beautifying and whitening agents, hair dyes, perfumes, alcohols, metal soaps, surfactants other than those of the invention, and so on.

Hereafter, specific examples thereof will be described. In particular, the surfactant of the present invention is effective in reducing the irritation of paraben compounds, which are antiseptics.

Skin-irritating substances:

Bacteriocidal antiseptics such as salicylic acid, paraben compounds (methylparaben, propylparaben, butylparaben, ethylparaben, etc.), hexachlorophene, imidazolidinylurea, quaternium-15, DM hydantoin, phenoxyethanol, and benzalkonium salts.

Sun screening agents such as p-aminobenzoic acid, antioxidants such as dibutylhydroxyltoluene, butylhydroxyanlsole and alkyl gallates, 2-hydroxy-4-methoxybenzophenone, para-amino acids such as octyldimethyl-p-aminobenzoate, and ethylhexyl-p-methoxycinnamate, organic ultraviolet absorbents such as hydroxybenzophenone-base, benzofuran-base, salicylic acid-base, coumarin-base, azole-base ultraviolet absorbents.

Ultraviolet rays reflection scattering agent such as titanium oxide, kaolin, and talc.

Vitamin agents such as vitamin A, C, E.

Pigments such as talc, kaolin, calcium carbonate, magnesium carbonate, magnesium silicate, silicic anhydride, titanium oxide, zinc oxide, red iron oxide, yellow iron oxide, chromium oxide, chromium hydroxide, carbon black, ultramarine, bismuth oxychloride, mica titanium mineral, and mica and organic and tar-base dyes such as butter yellow.

Beautifying and whitening agents such as kojic acid, albutin, mulberry root bark, placenta extract, SS albutin, ellagic acid, chamomile extract, and ascorbic acid derivatives.

Hair dyes such as oxidative dyes and acidic dyes and color aids, e.g., 5-amino-o-cresol, 2-amino-4-nitrophenol, 2-amino-5-nitrophenol, 1-amino-4-methylaminoanthraquinone, 3,3'-iminodiphenol, 2,4-diaminophenoxyethanol hydrochloride, 2,4-diaminophenol hydrochloride, toluene-2,5-diamine hydrochloride, nitro-p-phenylenediamine hydrochloride, p-phenylenediamine hydrochloride, N-phenyl-p-phenylenediamine hydrochloride, m-phenylenediamine hydrochloride, o-aminophenol, catechol, N-phenyl-p-phenylenediamine acetate, 1,4-diaminoanthraquinone, 2,6-diaminopyridine, 1,5-dihydroxynaphthalene, diphenylamine, ammonium carbonate, toluene-2,5-diamine, toluene-3,4-diamine, α-naphthol, nitro-p-phenylenediamine, p-aminophenol, p-nitro-o-phenylenediamine, p-phenylenediamine, p-methylaminophenol, picramic acid, sodium picramate, N,N-bis(4-aminophenyl)-2,5-diamino-1,4-quinone-diimine, 5-(2-hydroxyethylamino)-2-methylphenol, sodium 2-hdryoxy-5-nitro-2',4'-diaminoazobenzene-5-sulfonate, hydroquinone, pyrogallol, N-phenyl-p-phenylenediamine, phloroglucin, hematein, gallic acid, m-aminophenol, m-phenylenediamine, 5-amino-o-cresol sulfate, 2-amino-5-nitrophenol sulfate, o-aminophenol sulfate, o-chloro-p-phenylenediamine sulfate, 4,4'-diaminodiphenylamine sulfate, 2,4-diamminophenol sulfate, toluene-2,5-diamine sulfate, nitro-p-phenylenediamine sulfate, p-aminophenol sulfate, p-nitro-o-phenylenediamine sulfate, p-nitro-m-phenylenediamine sulfate, p-phenylenediamine sulfate, p-methylaminophenol sulfate, m-aminophenol sulfate, m-phenylenediamine sulfate, etc.

Perfumes such as sesquiterpene alcohol, geraniol, and linalool.

pH adjusting agent, buffer, or chelating agent, e.g., disodium edetate, ethylenediaminetetraacetic acid salts, pyrophosphoric acid salts, hexametaphosphoric acid salts, tartaric acid, gluconic acid, sodium hydroxide, triethanolamine, citric acid, sodium citrate, boric acid, borax, potassium hydrogen phosphate, etc.

Astringent such as zinc p-phenolsulfonate.

Alcohols such as ethanol and isopropanol.

Metal soaps such as magnesium, calcium and aluminum stearates, zinc laurate, and zinc palmitate.

In the external preparation for skin of the present invention, known synthetic surfactant may be used in combination and also the irritation of the surfactant can be reduced.

The surfactant in this case include, for example, nonionic surfactants such as oleophilic glycerin monostearate, self-emulsifying glycerin monostearate, polyglycerin monostearate, sorbitan monooleate, polyethylene glycol monostearate, polyoxyethylene sorbitan monooleate, polyoxyethylene cetyl ether, polyoxyethylenated sterol, polyoxyethylene alkyl ether, polyoxyethylene fatty acid ester, polyoxyethylene sorbitan fatty acid ester, polyoxyethylenated lanolin, poloxyethylenated beeswax, and polyoxyethylene-hardened castor oil, anionic surfactants such as sodium stearylphosphate, potassium palmitate, sodium cetylsulfate, sodium laurylphosphate, triethanolamine palmitate, sodium polyoxyethylene laurylphosphate and sodium N-acylglutaminate, cationic surfactants such as stearyldimethylbenzylammonium chloride and stearyltrimethylammonium chloride, amphoteric surfactants such as alkylaminoethylglycine hydrochloride liquid, and lecithin.

As other irritating substances, among oils and fats, oxidized lipids and lipid peroxides may be irritants. For example, it is when lipids as set forth below are oxidized.

There can be cited, for example, plant oils and fats such as castor oil, olive oil, cacao oil, camellia oil, coconut oil, haze wax, jojoba oil, grape seed oil, avogado oil, beefsteak plant oil, perilla oil, animal oils and fats such as mink oil, yolk oil, eicosapentaenoic acid (EPA), and docosahexaenoic acid (DHA), waxes such as beeswax, spermaceti, lanolin, carnauba wax, and candelilla wax, hydrocarbons such as liquid paraffin, squalane, microcrystalline wax, ceresin wax, paraffin wax, and vaseline, natural and synthetic fatty acids such as lauric acid, myristic acid, stearic acid, oleic acid, isostearic acid, and behenic acid, natural and synthetic higher alcohols such as cetanol, stearyl alcohol, hexyldecanol, octyldodecanol, and lauryl alcohol, esters such as isopropyl myristate, isopropyl palmitate, isopropyl adipate, octyldodecyl myristate, octyldodecyl oleate, and cholesterol oleate.

Also, permanent waving agent such as thioglycollic acid includes irritants.

In addition, there can be cited those substances which are converted into irritants during storage or use by physical, chemical or biological effects, for example, peroxides or various irritating decomposates. However, this invention is not limited thereto.

In addition to the above-described skin-irritating substances, the external preparation for skin of the present invention may contain usually used components such as surfactants, humectants, thickening agent, antiphlogistics, plant extract components, and other components as described below.

Examples of surfactant include sodium monofluorophosphate, fatty acid salts, alkylbenzenesulfonates, alkylnaphthalenesulfonates, alkylsulfonates, α-olefinsulfonates, dialkylsulfosuccinates, α-sulfonated fatty acid salts, alkylsulfates, polyoxyethylene alkyl ether sulfates, polyoxyethylene alkyl phenyl ether sulfates, polyoxyethylene styrenated phenyl ether sulfates, alkylphosphates, polyoxyethylene alkyl ether phosphates, polyoxyethylene alkyl phenyl ether phosphate, naphthalenesulfonate formalin condensate, polyoxyethylene alkyl ether, polyoxyethylene alkyl phenyl ether, polyoxyethylene polystyryl phenyl ether, polyoxyethylene polyoxypropylene glycol, polyoxyethylene polyoxypropylene alkyl ether, polyhydric alcohol fatty acid partial ester, polyoxyethylene polyhydric alcohol fatty acid partial ester, polyoxyethylene fatty acid ester, polyglycerin fatty acid ester, polyoxyethylenated castor oil, fatty acid diethanolamide, polyoxyethylene alkylamine, triethanolamine fatty acid partial ester, trialkylamine oxide, fatty acid amine salt, tetraalkylammonium salt, trialklbenzylammonium salt, alkylpyridinium salt, 2-alkyl-1-alkyl-1-hydroxyethylimidazolium salt, N,N-dialkylmorpholinium salt, polyethylene polyamine fatty acid amide salt, etc.

Examples of humectant includes polyhydric alcohols, such as glycerin, propylene glycol, 1,3-butylene glycol, sorbitol, polyglycerin, polyethylene glycol, and dipropylene glycol, natural moisturizing factor (NMF) components such as amino acids and sodium lactate, water-soluble polymers such as collagen, mucopolysaccharide and chondroitinsulfate, moisture-conditioner/humectants such as maltitol, sodium pyrolidonecarboxylate, polyoxyethylene methyl glucoside, hyaluronic acid, hyaluronic acid derivatives, ceramide, ceramide derivatives, ceramide analogues, and glucose.

Examples of thickening agent include natural polymers such as sodium alginate, xanthan gum, quince seed gum, tara gum, veegum, pectin, alginates, laponite, aluminum silicate, quince seed extract, tragacanth gum, and starch, semi-synthetic polymers such as methylcellulose, hydroxyethylcellulose, carboxymethylcellulose, soluble starch, and cationated cellulose, synthetic polymers such as acrylic polymer and polyvinyl alcohol, etc.

Antiphlogistics include salicylic acid derivative type antiphlogistics, aniline derivative type antiphlogistics, spasmolysants, pyrazolone derivative type antiphlogistics, indomethacin antiphlogistics, mephenamic acid antiphlogistics, anti-hisitamin agent, anti-allergy agent, anti-inflammatory enzyme agent, steroid agent, glycyrrhizin, azulene, allantoin, etc.

Combined use of these antiphlogistics will promote antiphlogistic effect on wound. Their usage may be generally on the order of 0.001 to 10 mol/l (external preparation for skin).

Plant extract components include triclosan (Irgasan-DP300 available from Ciba-Geigy Co., Ltd.), glycyrrhizic acid or its sodium or potassium salt or other salts, triethanolamine, hinoki extract, hinokithiol, edetates, propylene glycol, beefsteak plant extract, rosemary extract, rose extract, chamomile extract, Melissa extract, sage extract, licorice extract, jojoba extract, N-acyl-L-glutamic acid or its sodium salt or other salts, cetanol, *Sappiness mukorossi* extract, squalanes such as plant squalane, etc.

Other components include nutrients such as amino acids and amino acid derivatives, emolients such as ester oils and higher alcohols, abrasives such as calcium phosphate, aluminum hydroxide, calcium pyrophosphate, and insoluble sodium metaphosphate, ultraviolet absorbents, ultraviolet scattering agents, and those components which are described in the following raw material lists (1) to (8).

(1) Cosmetics raw material nonstandardized components standard (Yakuji Nippo, published October 14, 1993, pages 39-1368)
(2) Japan general use cosmetics raw material list, 2nd ed. (Yakuji Nippo, published March 25, 1989, pages 1-509)
(3) Japanese general use cosmetics raw material list, 3rd ed. (Yakuji Nippo, published June 30, 1994, pages 1-612)
(4) Medical drugs Japanese medicines, 21st ed. (Yakuji Nippo, published 1997, pages 1-2100)
(5) General drugs Japanese medicines, 1998-99 (Yakuji Nippo, November 10, 1997)
(6) 13th Revised Japan Pharmacopoeia First Supplement (Yakugyo Jiho, published January 31, 1998, pages 58-190)
(7) List of laws and ordinances relating to existing additives register associated with amendment of food hygiene law (edited by Food chemistry section, life hygiene bureau, Ministry of Commonwealth, published July 10, 1996, Social Insurance Publishers, pages 5-221)
(8) List of standards on the components of food additives, 8th ed. (Japan Science Feeding Stuff Association, published November 18, 1996, pages 7-827).

The surfactants, humectants, thickening agents, antiphlogistics, plant extracts components and other blending components may be added solely or in combination. There is no limitation on the addition amount thereof but usually they may be added in preparations in amounts in a range of 0.0001 to 80 wt%.

BEST MODE FOR CARRYING OUT THE INVENTION

Next, the present invention will be described in further detail by examples and formulations. However, the present invention should not be construed as being limited thereto. In the following examples, all "%" means "wt%" unless other indicated specifically as in the case of toxicity, for example.

Production Example

*Bacillus subtilis* ATCC 21332 strain was inoculated in a medium (1% polypeptone, 0.1% $KH_2PO_4$, 0.05% $MgSO_4 \cdot 7H_2O$, adjusted with NaOH to pH 7, balance water) and incubated at 35°C at 160 rpm for 12 hours. 100 ml of the culture medium was inoculated into a 2-liter fermenter charged with a medium containing soybean powder and maltose as main components and potassium hydrogen phosphate, magnesium sulfate, calcium salt, iron salt, and manganese salt as inorganic salts, and incubated at 35°C with stirring and strong aeration for 48 hours. During the incubation, caustic soda was added to maintain the medium at pH 7.0 to 7.5. After completion of the incubation, the medium was centrifuged to remove the bacteria cells and the resultant culture supernatant was collected. A portion of the culture supernatant was freeze-dried to obtain a dried medium preparation. The remaining culture supernatant was adjusted with hydrochloric acid to pH 2 to precipitate a surfactin fraction. The supernatant was removed by centrifugation and the surfactin fraction was dissolved in an acetone organic solvent. The resultant solution was passed through

ultrafiltration membrane resistant to organic solvents (Cefilt UF10,000, a ceramic membrane filter manufactured by Nippon Gaishi) to recover a liquid fraction, thereby removing high molecular weight impurities. Then, to the liquid fraction was added activated carbon (ϕ 20μm) to deodorize and decolorize it. Thereafter, the activated carbon was removed by filtration and the filtrate was concentrated to dryness in an evaporator. Then, the resulting solids were dissolved in water while adding caustic soda thereto to maintain the pH around 7. The resultant solution was freeze-dried to obtain purified surfactin sodium salt powder. The dried medium preparation and surfactin sodium salt were used in the following tests.

Surface tension tests

The dried medium preparation and purified surfactin sodium salt were each dissolved in water in an amount of 0.1 wt% and their ability of decreasing surface tension was tested. Surface tension was measured by a plate method (25°C) using an automatic surface tension meter CBVP-Z type manufactured by Kyowa Kaimen Kagaku Co., Ltd. Table 1 shows the results.

Table 1:

| Surface Tension | |
|---|---|
| Water | 72.1 mN/m |
| Aqueous solution of dried medium preparation (0.1 wt%) | 28.4 mN/m |
| Aqueous solution of purified surfactin sodium (0.1 wt%) | 27.6 mN/m |

Example 1: Skin irritation test of surfactant

Using a three-dimensional skin model (trade name: Three-dimensional cultured skin model, manufactured by Gunze), skin irritation tests were performed. As the test substances were used the surfactin sodium salt and dried medium preparation of the Production Example above, and SDS (sodium dodecylsulfate), Amisoft LS-11 (manufactured by Ajinomoto, hereafter, referred to as Amisoft). The test substances were adjusted with PBS (Phosphate Buffer Saline, pH 7) so that they were in various concentrations. To the thus-adjusted test substances was exposed the skin model for 1 hour. Thereafter, the test substances were washed and incubated for 16 hours in a medium attached to the above-described three-dimensional skin model. After the incubation, a solution of MTT (3-(4,5-dimethylthiazol-2-yl)-2,5-diphenyltetrazolium bromide) was added and pigments were extracted with isopropanol rendered acidic with hydrochloric acid, followed by measurement of absorbance at a wavelength of 570 nm. This value was called A. Also, as a control, similar operations were repeated without addition of test substances and absorbance at a wavelength of 570 nm was measured. This value was called B. The MTT solution was added to PBS and extraction was performed similarly. Absorbance of the extract measured at a wavelength of 570 nm was named C. From these values was calculated cytotoxicity. Calculation was made according to the following equation.

$$\text{Cytotoxicity (\%)} = (1-(A-c)/(B-C)) \times 100$$

Plotting the concentration of a test substance on the axis of abscissa and cytotoxicity on the axis of ordinate, a graph was obtained, from which the concentration of a test compound at 50% cytotoxicity was read. Whether this value is large or small indicates whether skin irritation is strong or weak. Table 2 shows concentrations at 50% cytotoxicity.

Table 2

| Surfactant | Concentration of surfactant at 50% cytotoxicity |
|---|---|
| Surfactin sodium salt | 24.4 % |
| Dried medium preparation | 32.8 % |
| Amisoft | 2.7 % |
| SDS | 0.2 % |

As shown in Table 2, it is apparent that the surfactin sodium salt and dried medium preparation of the inventive

product exhibited very low skin irritation as compared with Amisoft and SDS, respectively.

Example 2: Skin penetration test of skin-irritating substances

One (donor side) of two chambers separated by a hairless mouse skin was filled with a phosphate buffer (pH 7) having dissolved therein a substance having the composition described in Table 3 and the other (receiver side) was filled with a phosphate buffer solution (pH 7). After 5 hours, the concentration of methylparaben on the receiver side was measured. Table 3 shows the results. As will be apparent from the results, surfactin suppressed the skin penetration of skin-irritating substances.

Table 3

| Composition on donor side | Concentration of methylparaben of receiver |
|---|---|
| 0.1% Methylparaben | 5.7 ppm |
| 0.1% Methylparaben + 1% Surfactin sodium salt | 3.6 ppm |
| 0.1% Methylparaben + 1% dried medium preparation | 3.8 ppm |
| 0.1% Methylparaben + 1% SDS | 23.0 ppm |

Example 3: Skin irritation tests with external preparation for skin - 1

Milky lotions having the respective compositions shown in Table 4 were prepared by a conventional method and were evaluated for their skin irritation. In the same manner as in Example 1, a skin model was exposed to each of the prepared test substances (milky lotions described in Table 4) for 1 hour. Thereafter, the test substance was washed and incubated in the above-described medium for 16 hours. After the incubation, a MTT (3-(4,5-dimethylthiazol-2-yl)-2,5-diphenyltetrazolium bromide) was added, and pigments were extracted with isopropanol rendered acidic with hydrochloric acid, followed by measurement of absorbance and calculation of cytotoxicity. The cytotoxicity was calculated according to the equation described in Example 1.
Table 5 shows the cytotoxicity of each test substance.

Table 4

| Component(%) | Inventive preparation | | Comparative preparation | |
|---|---|---|---|---|
| | 1 | 2 | 1 | 2 |
| Surfactin sodium salt | 3.0 | — | - | - |
| Dried medium preparation | - | 3.0 | - | - |
| Amisoft | - | - | 3.0 | - |
| SDS | - | - | - | 3.0 |
| Avogado oil | 11.0 | 11.0 | 11.0 | 11.0 |
| Behenyl alcohol | 0.6 | 0.6 | 0.6 | 0.6 |
| Stearic acid | 0.4 | 0.4 | 0.4 | 0.4 |
| 1,3-Butylene glycol | 10.1 | 10.1 | 10.1 | 10.1 |
| Perfume | 0.4 | 0.4 | 0.4 | 0.4 |
| Purified water | Balance | Balance | Balance | Balance |

Table 5

| Test Substance | Cytotoxicity |
|---|---|
| Inventive preparation 1 | 0.0% |
| Inventive preparation 2 | 0.0% |
| Comparative preparation 1 | 72.5% |
| Comparative preparation 2 | 98.5% |

As will be apparent from the results in Table 5, the inventive preparation had very low irritation to the skin.

Example 4: Skin irritation tests with external preparation for skin - 2

One (donor side) of two chambers separated by a hairless mouse skin was filled with a cosmetic having the composition described in Table 6 and the other (receiver side) was filled with a phosphate buffer solution (pH 7). After 5 hours, the concentration of methylparaben on the receiver side was measured. Table 7 shows the results.

As will be apparent from the results, the inventive preparation showed a limited amount of skin penetration of methylparaben, a skin-irritating substance.

Table 6

| Component(%) | Inventive preparation 1 | Inventive preparation 2 | Comparative preparation |
|---|---|---|---|
| Surfactin sodium salt | 3.0 | - | - |
| Dried medium preparation | - | 3.0 | - |
| Ethyl alcohol | 39.6 | 39.6 | 39.6 |
| 1,3-Butylene glycol | 9.5 | 9.5 | 9.5 |
| Castor oil | 4.9 | 4.9 | 4.9 |
| Tocopherol | 1.0 | 1.0 | 1.0 |
| Methylparaben | 0.2 | 0.2 | 0.2 |
| Purified water | Balance | Balance | Balance |

Table 7

| Test Substance | Concentration of methylparaben |
|---|---|
| Inventive preparation 1 | 3.5 ppm |
| Inventive preparation 2 | 3.6 ppm |
| Comparative preparation | 7.0 ppm |

Example 5

Samples were prepared by dissolving surfactin sodium salt in a concentration of 0 or 0.2% and calcium chloride dihydrate in a calcium concentration of 0, 10, or 20 ppm in each of solvents described below, and each solution was charged in a screw vial and sealed, which was then left to stand at 40°C for 7 days. After 7 days, the turbidity of each sample was judged visually. Table 8 shows the results.

Solvent 1: Deionized water
Solvent 2: 7% Ethanol, 93% deionized water
Solvent 3: 7% Ethanol, 5% glycerin, 5% 1,3-butylene glycol, 83% deionized water

Table 8

| Composition of Samples | Concentration of calcium(ppm) | | |
|---|---|---|---|
| | 0 | 10 | 20 |
| Solvent 1 + Surfactin 0% | - | - | - |
| Solvent 2 + Surfactin 0% | - | - | - |
| Solvent 3 + Surfactin 0% | - | - | - |
| Solvent 1 + Surfactin 0.2% | - | + | + |
| Solvent 2 + Surfactin 0.2% | - | + | + |
| Solvent 3 + Surfactin 0.2% | - | + | + |
| -: Not turbid<br>+: Turbid | | | |

As will be apparent from the results in Table 8, in solvents containing surfactin sodium salts, turbidity occurs in the presence of calcium.

Example 6

Samples were prepared by dissolving 0.2% surfactin sodium salt and magnesium chloride dihydrate in a magnesium concentration of 0, 10, or 20 ppm in each of solvents described below, and each solution was charged in a screw vial and sealed, which was then left to stand at 40°C for 7 days. After 7 days, the turbidity of each sample was judged visually. Table 9 shows the results.

Solvent 1: Deionized water
Solvent 2: 7% Ethanol, 93% deionized water
Solvent 3: 7% Ethanol, 5% glycerin, 5% 1,3-butylene glycol, 83% deionized water

Table 9

| Composition of Samples | Concentration of magnesium(ppm) | | |
|---|---|---|---|
| | 0 | 10 | 20 |
| Solvent 1 | - | + | + |
| Solvent 2 | - | ± | ± |
| Solvent 3 | - | ± | ± |

-: Not turbid
±: Slightly turbid
+: Turbid

As will be apparent from the results in Table 9, in solvents containing surfactin sodium salts, turbidity occurs in the presence of magnesium.

Example 7

Samples were prepared by dissolving 0.2% surfactin sodium salt and calcium chloride dihydrate in a calcium concentration of 0.1, 1, or 10 ppm in the solvent described below and further disodium edetate was added in an amount of 0, 0.0001, 0.001, 0.01, 0.1, 0.2, 1, or 3%, respectively. Then, each solution was charged in a screw vial and sealed, which was then left to stand at 40°C for 7 days. After 7 days, the turbidity of each sample was judged visually. Table 10 shows the results.

Solvent: 7% Ethanol, 5% glycerin, 5% 1,3-butylene glycol, 83% deionized water.

Table 10

| Concentration of sodium edetate(%) | Concentration of calcium(ppm) | | |
|---|---|---|---|
| | 0.1 | 1 | 10 |
| 0 | - | + | + |
| 0.0001 | - | ± | + |
| 0.001 | - | - | + |
| 0.01 | - | - | - |
| 0.1 | - | - | - |
| 0.2 | - | - | - |
| 1 | - | - | - |
| 3 | - | - | - |

-: Not turbid
±: Slightly turbid
+: Turbid

As will be apparent from the results in Table 10, in solvents containing surfactin sodium salt and calcium, addition of disodium edetate prevented turbidity from occurring.

Example 8

Samples were prepared by dissolving 0.2% surfactin sodium salt and calcium chloride dihydrate in a calcium concentration of 0.1, 1, or 10 ppm in the solvent described below and further sodium citrate was added in an amount of 0, 0.0001, 0.001, 0.01, 0.1, 0.2, 1, or 3%, respectively. Then, each solution was charged in a screw vial and sealed, which was then left to stand at 40°C for 7 days. After 7 days, the turbidity of each sample was judged visually. Table 11 shows the results.

Solvent: 7% Ethanol, 5% glycerin, 5% 1,3-butylene glycol, 83% deionized water.

Table 11

| Concentration of sodium citrate(%) | Concentration of calcium(ppm) | | |
|---|---|---|---|
| | 0.1 | 1 | 10 |
| 0 | - | + | + |
| 0.0001 | - | ± | + |
| 0.001 | - | - | + |
| 0.01 | - | - | - |
| 0.1 | - | - | - |
| 0.2 | - | - | - |
| 1 | - | - | - |
| 3 | - | - | - |
| -: Not turbid<br>±: Slightly turbid<br>+: Turbid | | | |

As will be apparent from the results in Table 11, in solvents containing surfactin sodium salt and calcium, addition of sodium citrate is effective in inhibiting the occurrence of turbidity, similarly to disodium edetate.

Example 9

Milky lotions having the composition shown in Table 12 were prepared and were evaluated for their skin irritation. In the same manner as in Example 1, a skin model was exposed to each of the prepared test substances (milky lotions described in Table 12) for 1 hour. Thereafter, the test substance was washed and incubated in the above-described medium for 16 hours. After the incubation, a MTT (3-(4,5-dimethylthiazol-2-yl)-2,5-diphenyltetrazolium bromide) was added, and pigments were extracted with isopropanol rendered acidic with hydrochloric acid, followed by measurement of absorbance and calculation of cytotoxicity. The cytotoxicity was calculated according to the equation described in Example 1. Table 13 shows cytotoxicity of the test substances.

Table 12

| Component(%) | Inventive preparation 1 | Inventive preparation 2 | Inventive preparation 3 | Inventive preparation 4 | Comparative preparation 1 | Comparative preparation 2 |
|---|---|---|---|---|---|---|
| Surfactin sodium salt | 3.0 | - | 3.0 | - | 3.0 | - |
| Dried medium preparation | - | 3.0 | - | 3.0 | - | 3.0 |
| Disodium edetate | 0.01 | 0.01 | - | - | - | - |
| Sodium citrate | - | - | 0.01 | 0.01 | - | - |
| Avogado oil | 11.0 | 11.0 | 11.0 | 11.0 | 11.0 | 11.0 |
| Behenyl alcohol | 0.6 | 0.6 | 0.6 | 0.6 | 0.6 | 0.6 |
| Stearic acid | 0.4 | 0.4 | 0.4 | 0.4 | 0.4 | 0.4 |
| 1,3-Butylene glycol | 10.1 | 10.1 | 10.1 | 10.1 | 10.1 | 10.1 |
| Perfume | 0.4 | 0.4 | 0.4 | 0.4 | 0.4 | 0.4 |
| Purified water | Balance | Balance | Balance | Balance | Balance | Balance |

Table13

| Test Substance | Cytotoxicity |
|---|---|
| Inventive preparation 1 | 0.0 % |
| Inventive preparation 2 | 0.0 % |
| Inventive preparation 3 | 0.0 % |
| Inventive preparation 4 | 0.0 % |
| Comparative preparation 1 | 0.0 % |
| Comparative preparation 2 | 0.0 % |

As will be apparnt from the results shown in Table 13, the inventive preparation like the comparative preparations had very low sin irritation and the addition of disodium edetate or sodium citrate did not affect on the low irritation.

Example 10

One (donor side) of two chambers separated by a hairless mouse skin was filled with a cosmetic having the composition described in Table 14 and the other (receiver side) was filled with a phosphate buffer solution (pH 7). After 5 hours, the concentration of methylparaben on the receiver side was measured. Table 15 shows the results.

Table 14

| Component(%) | Inventive Preparation | | | | Comparative Preparation | | | | |
|---|---|---|---|---|---|---|---|---|---|
| | 1 | 2 | 3 | 4 | 1 | 2 | 3 | 4 | 5 |
| Surfactin sodium salt | 3.0 | - | 3.0 | - | - | - | 3.0 | - | - |
| Dried medium preparation | - | 3.0 | - | 3.0 | - | - | - | 3.0 | - |
| Disodium edetate | 0.01 | 0.01 | - | - | 0.01 | - | - | - | - |
| Sodium citrate | - | - | 0.01 | 0.01 | - | 0.01 | - | - | - |
| Ethyl alcohol | 39.6 | 39.6 | 39.6 | 39.6 | 39.6 | 39.6 | 39.6 | 39.6 | 39.6 |
| 1,3-Butylene glycol | 9.5 | 9.5 | 9.5 | 9.5 | 9.5 | 9.5 | 9.5 | 9.5 | 9.5 |
| Castor oil | 4.9 | 4.9 | 4.9 | 4.9 | 4.9 | 4.9 | 4.9 | 4.9 | 4.9 |
| α-Tocopherol | 1.0 | 1.0 | 1.0 | 1.0 | 1.0 | 1.0 | 1.0 | 1.0 | 1.0 |
| Methylparaben | 0.2 | 0.2 | 0.2 | 0.2 | 0.2 | 0.2 | 0.2 | 0.2 | 0.2 |
| Purified water | Balance | Balance | Balance | Balance | Balance | Balance | Balance | Balance | Balance |

Table 15

| Test Substance | Concentration of methylparaben |
|---|---|
| Inventive preparation 1 | 3.6 ppm |
| Inventive preparation 2 | 3.7 ppm |
| Inventive preparation 3 | 3.5 ppm |
| Inventive preparation 4 | 3.6 ppm |
| Comparative preparation 1 | 7.2 ppm |
| Comparative preparation 2 | 7.1 ppm |
| Comparative preparation 3 | 3.5 ppm |
| Comparative preparation 4 | 3.6 ppm |
| Comparative preparation 5 | 7.0 ppm |

As will be apparent from the results, the inventive preparation like the comparative preparations 3 and 4 suppressed the skin penetration of methylparaben, a skin-irritating substance. Therefore, it revealed that the addition of disodium edetate or sodium citrate did not affect on the effect of reducing the irritation of skin-irritating substances.

Formulation Examples

In the following formulation examples of external preparation for skin, APM and APS indicate magnesium ascorbic acid 2-phosphate and sodium ascorbic acid 2-phosphate. Also, "%" means "wt%".

Formulation Example 1: Beauty wash

The following components were dissolved with heating at 50°C and cooled with stirring until the temperature reached 30°C when the stirring was stopped and left to stand to prepare a beauty wash.

| | |
|---|---|
| Ethyl alcohol | 39.6% |
| 1,3-Butylene glycol | 9.5% |
| Castor oil | 4.9% |
| α-Tocopherol | 1.0% |
| Magnesium ascorbic 2-phosphate | 1.0% |
| Sodium ascorbic 2-phosphate | 1.0% |
| Methylparaben | 0.2% |
| Surfactin | 1.0% |
| Purified water | balance |

Formulation Example 2: Milky lotion

The following components were dissolved with heating at 80°C with stirring to emulsify. This was cooled with stirring and the stirring was stopped at 40°C, and the mixture was left to stand to prepare a milky lotion.

| | |
|---|---|
| Avogado oil | 11.0% |
| Behenyl alcohol | 0.6% |
| Stearic acid | 0.4% |
| Glycerin fatty acid ester | 0.9% |
| Polyoxyethylene sorbitan fatty acid ester | 1.1% |
| Polyoxyethylene alkyl ether | 0.4% |
| α-Tocopherol | 1.0% |
| Magnesium ascorbic 2-phosphate | 1.0% |
| Sodium ascorbic 2-phosphate | 1.0% |
| 1,3-Butylene glycol | 10.1% |
| Methylparaben | 0.2% |

(continued)

| | |
|---|---|
| Perfume | 0.4% |
| Surfactin | 0.5% |
| Purified water | balance |

Formulation Example 3: Cream

The following components were dissolved with heating at 80°C with stirring to emulsify. This was cooled with stirring and the stirring was stopped at 40°C, and the mixture was left to stand to prepare a cream.

| | |
|---|---|
| Squalane | 11.1% |
| Stearic acid | 7.8% |
| Stearyl alcohol | 6.0% |
| Beeswax | 1.9% |
| Propylene glycol monostearate | 3.1% |
| Polyoxyethylene cetyl ether | 1.1% |
| α-Tocopherol | 1.0% |
| Magnesium ascorbic 2-phosphate | 1.0% |
| Sodium ascorbic 2-phosphate | 1.0% |
| 1,3-Butylene glycol | 10.1% |
| Methylparaben | 0.2% |
| Perfume | 0.4% |
| Surfactin | 0.5% |
| Purified water | balance |

Formulation Example 4: Pack

The following components were swelled with heating at 50°C and uniformly mixed with stirring. This was cooled with stirring and the stirring was stopped at 30°C, and the mixture was left to stand to prepare a pack.

| | |
|---|---|
| Polyvinyl alcohol | 14.5% |
| Sodium carboxymethylcellulose | 4.8% |
| 1,3-Butylene glycol | 2.9% |
| α-Tocopherol | 1.0% |
| Magnesium ascorbic 2-phosphate | 1.0% |
| Sodium ascorbic 2-phosphate | 1.0% |
| Ethyl alcohol | 10.0% |
| Methylparaben | 0.1% |
| Surfactin | 0.5% |
| Purified water | balance |

Formulation Example 5: Lipstick

A red pigment was dispersed in castor oil using a roll mill to prepare a dispersion (A). To the dispersion were dissolved with heating other blending components in the following proportions and mixed well. The mixture was filtered and cast in a mold at a high temperature and cooled. The molded composition was charged in a vessel to prepare a lipstick.

| | |
|---|---|
| Castor oil | 45.3% |
| Hexadecyl alcohol | 25.2% |
| Lanoline | 3.9 |
| Beeswax | 4.8% |
| Ozocerite | 3.4% |

(continued)

| | |
|---|---|
| Candelilla wax | 6.2% |
| Carnauba wax | 2.1% |
| Methylparaben | 0.1% |
| α-Tocopherol | 1.0% |
| Magnesium ascorbic 2-phosphate | 1.0% |
| Sodium ascorbic 2-phosphate | 1.0% |
| Titanium oxide | 2.1% |
| Red pigment | 4.8% |
| Perfume | 0.1% |
| Surf act in | 0.1% |
| Moisture | balance |

Formulation Example 6: Beauty wash

The following components were dissolved with heating at 50°C and cooled with stirring. The stirring was stopped at 30°C, and the mixture was left to stand to prepare a beauty wash.

| | |
|---|---|
| Ethyl alcohol | 39.6% |
| 1,3-Butylene glycol | 9.5% |
| Castor oil | 4.9% |
| α-Tocopherol | 1.0% |
| APM or APS | 3.0% |
| Kojic acid | 1.0% |
| Placenta extract | 1.0% |
| Albutin | 1.0% |
| Citric acid | 0.5% |
| Tartaric acid | 0.5% |
| Malic acid | 0.5% |
| NaOH | (s. a. to make weakly alkaline pH) |
| Methylparaben | 0.2% |
| Surfactin | 0.5% |
| Purified water | balance |

Formulation Example 7: Beauty wash

The following components were dissolved with heating at 50°C and cooled with stirring. The stirring was stopped at 30°C, and the mixture was left to stand to prepare a beauty wash.

| | |
|---|---|
| Ethyl alcohol | 39.6% |
| 1,3-Butylene glycol | 9.5% |
| Castor oil | 4.9% |
| α-Tocopherol | 1.0% |
| APM or APS | 3.0% |
| Kojic acid | 1.0% |
| Placenta extract | 1.0% |
| Albutin | 1.0% |
| Citric acid | 0.5% |
| Tartaric acid | 0.5% |
| Malic acid | 0.5% |
| EDTA 2Na | 1.0% |
| NaOH | (s. a. to make weakly alkaline pH) |

(continued)

| | |
|---|---|
| Methylparaben | 0.2% |
| Surfactin | 0.5% |
| Purified water | balance |

Formulation Example 8: Beauty wash

The following components were dissolved with heating at 50°C and cooled with stirring. The stirring was stopped at 30°C, and the mixture was left to stand to prepare a beauty wash.

| | |
|---|---|
| Ethyl alcohol | 39.6% |
| 1,3-Butylene glycol | 9.5% |
| Castor oil | 4.9% |
| APM or APS | 3.0% |
| Methylparaben | 0.2% |
| Surfactin | 0.5% |
| Purified water | balance |

Formulation Example 9: Milky lotion

The following components were dissolved with heating at 80°C with stirring to emulsify. This was cooled with stirring and the stirring was stopped at 40°C, and the mixture was left to stand to prepare a milky lotion.

| | |
|---|---|
| Avogado oil | 11.0% |
| Behenyl alcohol | 0.6% |
| Stearic acid | 0.4% |
| Glycerin fatty acid ester | 0.9% |
| Polyoxyethylene sorbitan fatty acid ester | 1.1% |
| Polyoxyethylene alkyl ether | 0.4% |
| APM or APS | 3.0% |
| 1,3-Butylene glycol | 10.1% |
| Methylparaben | 0.2% |
| Perfume | 0.4% |
| Surfactin | 0.5% |
| Purified water | balance |

Formulation Example 10: Cream

The following components were dissolved with heating at 80°C with stirring to emulsify. This was cooled with stirring and the stirring was stopped at 40°C, and the mixture was left to stand to prepare a cream.

| | |
|---|---|
| Squalane | 11.1% |
| Stearic acid | 7.8% |
| Stearyl alcohol | 6.0% |
| Beeswax | 1.9% |
| Propylene glycol monostearate | 3.1% |
| Polyoxyethylene cetyl ether | 1.1% |
| APM or APS | 3.0% |
| 1,3-Butylene glycol | 11.9% |
| Methylparaben | 0.2% |
| Perfume | 0.4% |
| Surfactin | 1.0% |
| Purified water | balance |

Formulation Example 11: Pack

The following components were swelled with heating at 50°C and uniformly mixed with stirring. This was cooled with stirring and the stirring was stopped at 30°C, and the mixture was left to stand to prepare a pack.

| | |
|---|---|
| Polyvinyl alcohol | 14.5% |
| Sodium carboxymethylcellulose | 4.8% |
| 1,3-Butylene glycol | 2.9% |
| APM or APS | 3.0% |
| Ethyl alcohol | 10.0% |
| Ethylparaben | 0.1% |
| Surfactin | 0.1% |
| Purified water | balance |

Formulation Example 12: Lipstick

A red pigment was dispersed in castor oil using a roll mill to prepare a dispersion (A). To the dispersion were dissolved with heating other blending components in the following proportions and mixed well. The mixture was filtered and cast in a mold at a high temperature and cooled. The molded composition was charged in a vessel to prepare a lipstick.

| | |
|---|---|
| Castor oil | 45.3% |
| Hexadecyl alcohol | 25.2% |
| Lanoline | 3.9% |
| Beeswax | 4.8% |
| Ozocerite | 3.4% |
| Candelilla wax | 6.2% |
| Carnauba wax | 2.1% |
| Methylparaben | 0.1% |
| APM or APS | 3.0% |
| Titanium oxide | 2.1% |
| Red pigment | 4.8% |
| Perfume | 0.1% |
| Surfactin | 0.1% |
| Moisture | balance |

Formulation Example 13: Foundation

The following components were mixed at 80°C with stirring and then left to cool to prepare a foundation.

| | |
|---|---|
| Liquid paraffin | 23.5% |
| Isopropyl palmitate | 14.3% |
| Lanoline alcohol | 1.8% |
| Lanoline acetate | 2.9% |
| Microcrystalline wax | 6.5% |
| Ozocerite | 7.7% |
| Candelilla wax | 0.4% |
| Methylparaben | 0.1% |
| APM or APS | 3.0% |
| Titanium oxide | 14.5% |
| Kaolin | 13.9% |
| Talc | 5.7% |
| Coloring pigment | 3.9% |

(continued)

| | |
|---|---|
| Perfume | 0.5% |
| Surfactin | 0.1% |
| Moisture | balance |

Formulation Example 14: Dentifrice

The following compositions were swelled with heating, mixed well, and then left to stand to prepare a dentifrice composition.

| | |
|---|---|
| Calcium secondary phosphate dihydrate | 45.5% |
| Sodium carboxymethylcellulose | 0.5% |
| Carrageenan | 0.5% |
| Glycerin | 9.8% |
| Sorbitol | 9.7% |
| Sodium saccharinate | 0.1% |
| Surfactin | 2.0% |
| Sodium chloride | 2.1% |
| α-Tocopherol | 0.4% |
| APM or APS | 3.0% |
| Antiseptic | 0.1% |
| Perfume | 0.5% |
| Purified water | balance |

Formulation Example 15: Gargle

The following components were uniformly mixed at ambient temperature to prepare a gargle.

| | |
|---|---|
| Ethyl alcohol | 34.6% |
| Glycerin | 14.5% |
| α-Tocopherol | 0.4% |
| APM or APS | 3.0% |
| Surfactin | 0.1% |
| Perfume | 0.5% |
| Purified water | balance |

Formulation Example 16: Hair tonic

The following components were uniformly mixed at ambient temperature to prepare a hair tonic.

| | |
|---|---|
| Ethyl alcohol | 63.0% |
| Castor oil | 4.3% |
| Resorcinol | 0.7% |
| Methylparaben | 0.1% |
| Capsicum tincture | 0.4% |
| α-Tocopherol | 0.4% |
| APM or APS | 3.0% |
| Surfactin | 0.2% |
| Purified water | balance |

Formulation Example 17: Shampoo

The following components were dissolved by heating at 70°C and mixed with stirring. Then this was cooled

with stirring to 40°C and left to stand to prepare a shampoo.

| | |
|---|---|
| Triethanolamine laurylsulfate | 15.0% |
| Diethanolamide laurate | 3.3% |
| Triethanolamine polyacrylate | 0.3% |
| Zinc pyridinium-1-thiol-N-oxide | 1.1% |
| APM or APS | 3.0% |
| Surfactin | 1.0% |
| Pigment | minute amount |
| Perfume | 0.5% |
| Purified water | balance |

Formulation Example 18: Rinse

The following components were dissolved by heating at 80°C and mixed with stirring. Then this was cooled with stirring to 40°C and left to stand to prepare a rinse.

| | |
|---|---|
| Stearyldimethylbenzylammonium chloride | 1.4% |
| Stearyl alcohol | 0.6% |
| Glyceryl monostearate | 1.5% |
| Sodium chloride | 0.2% |
| APM or APS | 3.0% |
| Surfactin | 0.1% |
| Purified water | balance |

Formulation Example 19: Bath agent

The following components were uniformly mixed at ambient temperature to prepare a bath agent.

| | |
|---|---|
| Sodium hydrogen carbonate | 35.5% |
| Citric acid | 37.1% |
| Polyethylene glycol | 2.1% |
| Magnesium chloride | 1.1% |
| $\alpha$-Tocopherol | 0.5% |
| APM or APS | 24.0% |
| Surfactin | 1.0% |
| Pigment | minute amount |
| Perfume | 2.0% |

Formulation Example 20: Beauty wash

The following components were dissolved with heating at 50°C and cooled with stirring. The stirring was stopped at 30°C, and the mixture was left to stand to prepare a beauty wash.

| | |
|---|---|
| Ethyl alcohol | 39.6% |
| 1,3-Butylene glycol | 9.5% |
| Castor oil | 4.9% |
| $\alpha$-Tocopherol | 1.0% |
| Magnesium ascorbic 2-phosphate | 1.0% |
| Sodium ascorbic 2-phosphate | 1.0% |
| Methylparaben | 0.2% |
| Surfactin | 1.0% |
| Disodium edetate | 0.01% |

(continued)

| | |
|---|---|
| Purified water | balance |

Formulation Example 21: Milky lotion

The following components were dissolved with heating at 80°C with stirring to emulsify. This was cooled with stirring and the stirring was stopped at 40°C, and the mixture was left to stand to prepare a milky lotion.

| | |
|---|---|
| Avogado oil | 11.0% |
| Behenyl alcohol | 0.6% |
| Stearic acid | 0.4% |
| Glycerin fatty acid ester | 0.9% |
| Polyoxyethylene sorbitan fatty acid ester | 1.1% |
| Polyoxyethylene alkyl ether | 0.4% |
| α-Tocopherol | 1.0% |
| Magnesium ascorbic 2-phosphate | 1.0% |
| Sodium ascorbic 2-phosphate | 1.0% |
| 1,3-Butylene glycol | 10.1% |
| Methylparaben | 0.2% |
| Perfume | 0.4% |
| Surfactin | 0.5% |
| Disodium edetate | 0.01% |
| Purified water | balance |

Formulation Example 22: Cream

The following components were dissolved with heating at 80°C with stirring to emulsify. This was cooled with stirring and the stirring was stopped at 40°C, and the mixture was left to stand to prepare a cream.

| | |
|---|---|
| Squalane | 11.1% |
| Stearic acid | 7.8% |
| Stearyl alcohol | 6.0% |
| Beeswax | 1.9% |
| Propylene glycol monostearate | 3.1% |
| Polyoxyethylene cetyl ether | 1.1% |
| α-Tocopherol | 1.0% |
| Magnesium ascorbic 2-phosphate | 1.0% |
| Sodium ascorbic 2-phosphate | 1.0% |
| 1,3-Butylene glycol | 10.1% |
| Methylparaben | 0.2% |
| Perfume | 0.4% |
| Surfactin | 0.5% |
| Sodium citrate | 0.01% |
| Purified water | balance |

Formulation Example 23: Pack

The following components were swelled with heating at 50°C and uniformly mixed with stirring. This was cooled with stirring and the stirring was stopped at 30°C, and the mixture was left to stand to prepare a pack.

| | |
|---|---|
| Polyvinyl alcohol | 14.5% |
| Sodium carboxymethylcellulose | 4.8% |

(continued)

| | |
|---|---|
| 1,3-Butylene glycol | 2.9% |
| α-Tocopherol | 1.0% |
| Magnesium ascorbic 2-phosphate | 1.0% |
| Sodium ascorbic 2-phosphate | 1.0% |
| Ethyl alcohol | 10.0% |
| Methylparaben | 0.1% |
| Surfactin | 0.5% |
| Sodium citrate | 0.01% |
| Purified water | balance |

Formulation Example 24: Lipstick

A red pigment was dispersed in castor oil using a roll mill to prepare a dispersion (A). To the dispersion were dissolved with heating other blending components in the following proportions and mixed well. The mixture was filtered and cast in a mold at a high temperature and cooled. The molded composition was charged in a vessel to prepare a lipstick.

| | |
|---|---|
| Castor oil | 45.3% |
| Hexadecyl alcohol | 25.2% |
| Lanoline | 3.9% |
| Beeswax | 4.8% |
| Ozocerite | 3.4% |
| Candelilla wax | 6.2% |
| Carnauba wax | 2.1% |
| Methylparaben | 0.1% |
| α-Tocopherol | 1.0% |
| Magnesium ascorbic 2-phosphate | 1.0% |
| Sodium ascorbic 2-phosphate | 1.0% |
| Titanium oxide | 2.1% |
| Red pigment | 4.8% |
| Perfume | 0.1% |
| Surfactin | 0.1% |
| Disodium edetate | 0.005% |
| Moisture | balance |

Formulation Example 25: Beauty wash

The following components were dissolved with heating at 50°C and cooled with stirring. The stirring was stopped at 30°C, and the mixture was left to stand to prepare a beauty wash.

| | |
|---|---|
| Ethyl alcohol | 39.6% |
| 1,3-Butylene glycol | 9.5% |
| Castor oil | 4.9% |
| APM or APS | 3.0% |
| Sodium citrate | 0.1% |
| Methylparaben | 0.2% |
| Surfactin | 0.5% |
| Purified water | balance |

Formulation Example 26: Milky lotion

The following components were dissolved with heating at 80°C with stirring to emulsify. This was cooled with

stirring and the stirring was stopped at 40°C, and the mixture was left to stand to prepare a milky lotion.

| | |
|---|---|
| Avogado oil | 11.0% |
| Behenyl alcohol | 0.6% |
| Stearic acid | 0.4% |
| Glycerin fatty acid ester | 0.9% |
| Polyoxyethylene sorbitan fatty acid ester | 1.1% |
| Polyoxyethylene alkyl ether | 0.4% |
| APM or APS | 3.0% |
| 1,3-Butylene glycol | 10.1% |
| Disodium edetate | 0.01% |
| Methylparaben | 0.2% |
| Perfume | 0.4% |
| Surfactin | 0.5% |
| Purified water | balance |

Formulation Example 27: Cream

The following components were dissolved with heating at 80°C with stirring to emulsify. This was cooled with stirring and the stirring was stopped at 40°C, and the mixture was left to stand to prepare a cream.

| | |
|---|---|
| Squalane | 11.1% |
| Stearic acid | 7.8% |
| Stearyl alcohol | 6.0% |
| Beeswax | 1.9% |
| Propylene glycol monostearate | 3.1% |
| Polyoxyethylene cetyl ether | 1.1% |
| APM or APS | 3.0% |
| 1,3-Butylene glycol | 11.9% |
| Disodium edetate | 0.01% |
| Methylparaben | 0.2% |
| Perfume | 0.4% |
| Surfactin | 1.0% |
| Purified water | balance |

Formulation Example 28: Pack

The following components were swelled with heating at 50°C and uniformly mixed with stirring. This was cooled with stirring and the stirring was stopped at 30°C, and the mixture was left to stand to prepare a pack.

| | |
|---|---|
| Polyvinyl alcohol | 14.5% |
| Sodium carboxymethylcellulose | 4.8% |
| 1,3-Butylene glycol | 2.9% |
| APM or APS | 3.0% |
| Ethyl alcohol | 10.0% |
| Disodium edetate | 0.001% |
| Ethylparaben | 0.1% |
| Surfactin | 0.1% |
| Purified water | balance |

Formulation Example 29: Lipstick

A red pigment was dispersed in castor oil using a roll mill to prepare a dispersion (A). To the dispersion were

dissolved with heating other blending components in the following proportions and mixed well. The mixture was filtered and cast in a mold at a high temperature and cooled. The molded composition was charged in a vessel to prepare a lipstick.

| | |
|---|---|
| Castor oil | 45.3% |
| Hexadecyl alcohol | 25.2% |
| Lanoline | 3.9% |
| Beeswax | 4.8% |
| Ozocerite | 3.4% |
| Candelilla wax | 6.2% |
| Carnauba wax | 2.1% |
| Methylparaben | 0.1% |
| APM or APS | 3.0% |
| Titanium oxide | 2.1% |
| Red pigment | 4.8% |
| Perfume | 0.1% |
| Edetic acid | 0.001% |
| Surfactin | 0.1% |
| Moisture | balance |

Formulation Example 30: Foundation

The following components were mixed at 80°C with stirring and then left to cool to prepare a foundation.

| | |
|---|---|
| Liquid paraffin | 23.5% |
| Isopropyl palmitate | 14.3% |
| Lanoline alcohol | 1.8% |
| Lanoline acetate | 2.9% |
| Microcrystalline wax | 6.5% |
| Ozocerite | 7.7% |
| Candelilla wax | 0.4% |
| Methylparaben | 0.1% |
| APM or APS | 3.0% |
| Titanium oxide | 14.5% |
| Kaolin | 13.9% |
| Talc | 5.7% |
| Coloring pigment | 3.9% |
| Perfume | 0.5% |
| Disodium edetate | 0.001% |
| Surfactin | 0.1% |
| Moisture | balance |

Formulation Example 31: Dentifrice

The following compositions were swelled with heating, mixed well, and then left to stand to prepare a dentifrice composition.

| | |
|---|---|
| Calcium secondary phosphate dihydrate | 45.5% |
| Sodium carboxymethylcellulose | 0.5% |
| Carrageenan | 0.5% |
| Glycerin | 9.8% |
| Sorbitol | 9.7% |
| Sodium saccharinate | 0.1% |

(continued)

| | |
|---|---|
| Surfactin | 2.0% |
| Sodium chloride | 2.1% |
| α-Tocopherol | 0.4% |
| APM or APS | 3.0% |
| Disodium edetate | 0.1% |
| Antiseptic | 0.1% |
| Perfume | 0.5% |
| Purified water | balance |

Formulation Example 32: Gargle

The following components were uniformly mixed at ambient temperature to prepare a gargle.

| | |
|---|---|
| Ethyl alcohol | 34.6% |
| Glycerin | 14.5% |
| α-Tocopherol | 0.4% |
| APM or APS | 3.0% |
| Sodium citrate | 0.01% |
| Surfactin | 0.1% |
| Perfume | 0.5% |
| Purified water | balance |

Formulation Example 33: Hair tonic

The following components were uniformly mixed at ambient temperature to prepare a hair tonic.

| | |
|---|---|
| Ethyl alcohol | 63.0% |
| Castor oil | 4.3% |
| Resorcinol | 0.7% |
| Methylparaben | 0.1% |
| Capsicum tincture | 0.4% |
| α-Tocopherol | 0.4% |
| APM or APS | 3.0% |
| Disodium edetate | 0.01% |
| Surfactin | 0.2% |
| Purified water | balance |

Formulation Example 34: Shampoo

The following components were dissolved by heating at 70°C and mixed with stirring. Then this was cooled with stirring to 40°C and left to stand to prepare a shampoo.

| | |
|---|---|
| Triethanolamine laurylsulfate | 15.0% |
| Diethanolamide laurate | 3.3% |
| Triethanolamine polyacrylate | 0.3% |
| Zinc pyridinium-1-thiol-N-oxide | 1.1% |
| APM or APS | 3.0% |
| Disodium edetate | 0.05% |
| Surfactin | 1.0% |
| Pigment | minute amount |
| Perfume | 0.5% |

(continued)

| | |
|---|---|
| Purified water | balance |

Formulation Example 35: Rinse

The following components were dissolved by heating at 80°C and mixed with stirring. Then this was cooled with stirring to 40°C and left to stand to prepare a rinse.

| | |
|---|---|
| Stearyldimethylbenzylammonium chloride | 1.4% |
| Stearyl alcohol | 0.6% |
| Glyceryl monostearate | 1.5% |
| Sodium chloride | 0.2% |
| APM or APS | 3.0% |
| Disodium edetate | 0.001% |
| Surfactin | 0.1% |
| Purified water | balance |

Formulation Example 36: Bath agent

The following components were uniformly mixed at ambient temperature to prepare a bath agent.

| | |
|---|---|
| Sodium hydrogen carbonate | 35.5% |
| Citric acid | 37.1% |
| Polyethylene glycol | 2.1% |
| Magnesium chloride | 1.1% |
| α-Tocopherol | 0.5% |
| APM or APS | 24.0% |
| Disodium edetate | 1.0% |
| Surfactin | 0.2% |
| Pigment | minute amount |
| Perfume | 2.0% |

INDUSTRIAL APPLICABILITY

The surfactant for use in external preparations for skin of the present invention has low skin penetration and low skin irritation. Therefore, they can be applied to cosmetics. Cosmetics usually contain skin-irritating substances and addition of the surfactant of the present invention can reduce the irritation of the irritating substances.

Surfactin, a typical example of the surfactant for use in external preparations for skin, can be produced by utilizing microbes so that it is advantageous from the viewpoint of production method.

Further, the external preparation for skin containing a sequestering agent according to the present invention can suppress the generation of turbidity in cosmetics attributable to the surfactant for use in external preparations for skin and alkaline earth metal and can retain transparency of cosmetics. Therefore, it is very advantageous in its application to transparent cosmetics.

## Claims

**1.** The use of at least one lipopeptide represented by formula (2) below or its salt

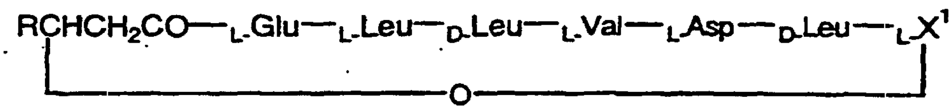

(wherein $X^1$ is an amino acid selected from the group consisting of leucine, isoleucine, valine, glycine, serine, alanine, threonine, asparagine, glutamine, aspartic acid, glutamic acid, lysine, arginine, cystein, methionine, phenylalanine, tyrosine, tryptophan, histidine, proline, 4-hydroxyproline, and homoserine, and R has 9 to 13 carbon atoms and is a n-alkyl group, an isoalkyl group, or an anteisoalkyl group), as a surfactant in external preparations for the skin.

**2.** The use according to claim 1, wherein $X^1$ is leucine, isoleucine or valine.

**3.** The use according to claim 1, wherein the surfactant is plipastatin, arthrofactin, iturin, or serrawettin.

**4.** The use according to any of the claims 1 to 3, wherein the surfactant is used for inhibiting the skin penetration of a skin-irritating substance.

**5.** The use according to any of the claims 1 to 3, wherein the surfactant is used for inhibiting the skin penetration of a skin-irritating substance and reduces irritation of a skin-irritating substance.

**6.** The use according to claim 5, wherein the skin-irritating substance is an antiseptic.

**7.** The use according to claim 6, wherein the antiseptic is a paraben compound.

**8.** The use according to any of the claims 1 to 7, wherein the surfactant is used in external preparations for the skin in a content of 0.01 to 30 wt.%.

**9.** The use according to any of the claims 1 to 8, wherein the external preparation for the skin further comprises a sequestering agent.

**10.** The use according to claim 9, wherein the surfactant is used in external preparations for skin in a content of 0.01 to 30 wt.% and the sequestering agent is used in a content of 0.0001 to 30 wt.%.

**11.** The use according to any of the claims 1 to 10, wherein the external preparation for the skin is a cosmetic.

**Patentansprüche**

**1.** Verwendung mindestens eines durch die Formel (2) unten dargestellten Lipopeptids oder dessen Salzes

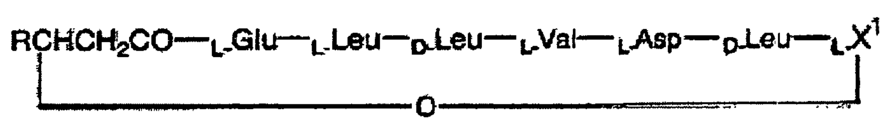

(worin $X^1$ eine Aminosäure ist, die aus der Gruppe ausgewählt ist, die aus Leucin, Isoleucin, Valin, Glycin, Serin, Alanin, Threonin, Asparagin, Glutamin, Asparaginsäure, Glutaminsäure, Lysin, Arginin, Cystein, Methionin, Phenylalanin, Tyrosin, Tryptophan, Histidin, Prolin, 4-Hydroxyprolin und Homoserin besteht, und R 9 bis 13 Kohlenstoffatome aufweist und eine n-Alkylgruppe, eine Isoalkylgruppe oder eine Anteisoalkylgruppe ist), als oberflächenaktives Mittel in externen Zubereitungen für die Haut.

**2.** Verwendung nach Anspruch 1, worin $X^1$ Leucin, Isoleucin oder Valin ist.

**3.** Verwendung nach Anspruch 1, worin das oberflächenaktive Mittel Plipastatin, Arthrofactin, Iturin oder Serrawettin ist.

**4.** Verwendung nach einem der Ansprüche 1 bis 3, worin das oberflächenaktive Mittel dazu verwendet wird, das Eindringen einer hautreizenden Substanz in die Haut zu hemmen.

**5.** Verwendung nach einem der Ansprüche 1 bis 3, worin das oberflächenaktive Mittel dazu verwendet wird, das Eindringen einer hautreizenden Substanz in die Haut zu hemmen und die Reizung einer hautreizenden Substanz zu vermindern.

**6.** Verwendung nach Anspruch 5, worin die hautreizende Substanz ein Antiseptikum ist.

**7.** Verwendung nach Anspruch 6, worin das Antiseptikum eine Parabenverbindung ist.

**8.** Verwendung nach einem der Ansprüche 1 bis 7, worin das oberflächenaktive Mittel in externen Zubereitungen für die Haut in einem Gehalt von 0,01 bis 30 Gew.-% verwendet wird.

**9.** Verwendung nach einem der Ansprüche 1 bis 8, worin die externe Zubereitung für die Haut weiterhin ein Sequestrierungsmittel umfaßt.

**10.** Verwendung nach Anspruch 9, worin das oberflächenaktive Mittel in externen Zubereitungen für die Haut in einem Gehalt von 0,01 bis 30 Gew.-% verwendet wird, und das Sequestrierungsmittel in einem Gehalt von 0,0001 bis 30 Gew.-% verwendet wird.

**11.** Verwendung nach einem der Ansprüche 1 bis 10, worin die externe Zubereitung für die Haut ein Kosmetikum ist.

**Revendications**

**1.** Utilisation d'au moins un lipopeptide représenté par la formule (2) ci-dessous ou son sel

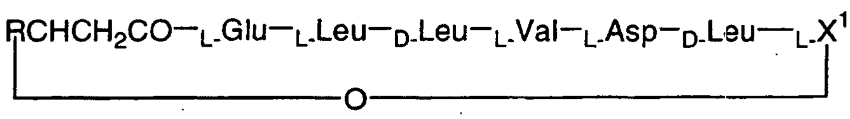

(dans laquelle $X^1$ est un acide aminé choisi dans le groupe constitué par la leucine, l'isoleucine, la valine, la glycine, la sérine, l'alanine, la thréonine, l'asparagine, la glutamine, l'acide aspartique, l'acide glutamique, la lysine, l'arginine, la cystéine, la méthionine, la phénylalanine, la tyrosine, le tryptophane, l'histidine, la proline, la 4-hydroxyproline, et l'homosérine, et R a 9 à 13 atomes de carbone et est un groupe n-alkyle, un groupe isoalkyle, ou un groupe antéisoalkyle), comme tensioactif dans des préparations externes pour la peau.

**2.** Utilisation selon la revendication 1, dans laquelle $X^1$ est la leucine, l'isoleucine ou la valine.

**3.** Utilisation selon la revendication 1, dans laquelle le tensioactif est la plipastatine, l'arthrofactine, l'iturine, ou la serrawettine.

**4.** Utilisation selon l'une quelconque des revendications 1 à 3, dans laquelle le tensioactif est utilisé pour inhiber la pénétration par la peau d'une substance irritant la peau.

**5.** Utilisation selon l'une quelconque des revendications 1 à 3, dans laquelle le tensioactif est utilisé pour inhiber la pénétration par la peau d'une substance irritant la peau et réduit l'irritation d'une substance irritant la peau.

**6.** Utilisation selon la revendication 5, dans laquelle la substance irritant la peau est un antiseptique.

**7.** Utilisation selon la revendication 6, dans laquelle l'antiseptique est un composé parabène.

8. Utilisation selon l'une quelconque des revendications 1 à 7, dans laquelle le tensioactif est utilisé dans des préparations externes pour la peau en une quantité de 0,01 à 30 % en poids.

9. Utilisation selon l'une quelconque des revendications 1 à 8, dans laquelle la préparation externe pour la peau comprend en outre un agent séquestrant.

10. Utilisation selon la revendication 9, dans laquelle le tensioactif est utilisé dans des préparations externes pour la peau en une quantité de 0,01 à 30 % en poids et l'agent séquestrant est utilisé en une quantité de 0,0001 à 30 % en poids.

11. Utilisation selon l'une quelconque des revendications 1 à 10, dans laquelle la préparation externe pour la peau est un cosmétique.